# EUROPEAN PATENT APPLICATION

(11) **EP 2 722 050 A2**
(43) Date of publication of application: **23.04.2014**
(21) Application number: 12800653.3
(22) Date of filing: 15.06.2012
(51) Int. Cl.: A61K 36/63, A61K 36/18, A61P 17/00

(54) **USE OF OLIVE OIL TO PREVENT AND TO TREAT DISEASES INVOLVING AN ISCHEMIC PROCESS**

(30) Priority: 16.06.2011 ES 201131012
(71) Applicant: Agencia Pública Empresarial Sanitaria Hospital Alto Guadalquivir, 23740 Andújar (Jaén) (ES); Servicio Andaluz de Salud, 41071 Sevilla (ES)
(72) Inventor: DÍAZ VALENZUELA, Antonio, E-41092 Sevilla (ES); VALLE CAÑETE, María Jesús, E-41092 Sevilla (ES); RUIZ GUTIÉRREZ, David, E-41092 Sevilla (ES); CARMONA FERNÁNDEZ, Pedro Jesús, E-41092 Sevilla (ES)
(74) Representative: HOFFMANN EITLE
(86) International application number: PCT/ES2012/070447
(87) International publication number: WO 2012/172150

(57) **Abstract**

Use of olive oil, preferably extra virgin olive oil, of its pharmaceutical forms and preparations, for producing a medicament for the prevention and treatment of diseases involving ischemic processes, and particularly pressure ulcers, vascular ulcers and diabetic foot neuropathic ulcers.

## Description

The present invention pertains to the field of medicine and pharmacy, and it more specifically refers to the use of olive oil, preferably extra virgin olive oil, for producing a medicament for the prevention and treatment of diseases involving an ischemic process, more specifically pressure ulcers, vascular ulcers and diabetic foot neuropathic ulcers. The invention also refers to a new composition comprising olive oil, hypericum, tocopherol and additionally mint as a cosmetic composition and as pharmaceutical composition, for producing a medicament for the prevention and treatment of diseases involving an ischemic process, and specifically pressure ulcers, vascular ulcers, and diabetic foot neuropathic ulcers.

### STATE OF THE ART

Ischemia, or tissue damage due to arterial insufficiency, is frequent in diabetic persons as a consequence of the damage suffered by their blood vessels because of the disease. A function of the arteries is to provide nutrients and oxygen to the cells, thus allowing for their correct operation. Blood supply to the foot is difficult due to the large distance to the heart; this, added to damaged blood vessels may cause an important reduction of arterial blood flow to the foot.

According to the definition given by the International Consensus on the Diabetic Foot, a "diabetic foot" is an infection, ulceration or destruction of the deep tissues connected with neurologic alterations and different degrees of peripheral vascular disease in the lower extremities affecting patients with diabetes mellitus.

It is important to stress that the "diabetic foot" should not mistakenly be taken by the foot of a diabetic person, since not all diabetic persons develop this complication, which greatly depends on how well the disease is controlled, on intrinsic and environmental factors associated with the patient, and finally on the evolution of the original base pathology.

With respect to the factors causing diabetic foot, it has been shown that in a dry and weak skin, due to blood flow insufficiency and lack of sensibility by the patient, a traumatism, such as a hit, a pointed or sharp object, a small stone, or even shoes that rub, may cause the disease.

A wound is thus created, and at the beginning the diabetic person would not even notice due to the lack of sensibility. This wound, since not treated, is an open door for microorganisms causing an infection. Since the inflammatory response is diminished, the pathogens do not find much resistance; they colonize the area, the tissue is destroyed, and cicatrizing becomes very difficult. An ulceration is thus created. Ulcers are wounds characterized by loss of substance and specially by a very low tendency towards cicatrization.

Specialists are currently studying the role of nitric oxide in the cicatrization of a wound of the diabetic foot. Nitric oxide, already employed in the art, is a powerful vasodilator which aids in conveying nutrients to oxygen lacking wounds. In 2004, a report was made whose conclusion was that, for persons having diabetic foot, hyperbaric oxygen therapy (medical use of oxygen at a pressure higher than atmospheric pressure) could improve cicatrization in a year. However, sometimes the general treatment is not appropriate for certain patients, and an individual evaluation must be carried out before indicating an specific treatment, even indicating a combination of different treatments if it is considered necessary.

Pressure ulcers are an important problem for the public health system, and at the same time a great challenge for the nursing activity in view of the repercussions in connection with the health and the quality of life of people suffering them, social and labor repercussions on the people taking care of patients, resource consumption, and a current increase in legal claims connected with prevention and treatment of pressure ulcers.

According to the most recent epidemiologic reports in our country, the mean prevalence in Primary Attention was 9,11% ± 10,9% for patients older than 14 years old included in the home medical attention program; in hospital units, the mean prevalence was 8,91% ± 12,20, and in socio-sanitary centers the mean prevalence was 10,9% ± 11,9%. With respect to teh economic impact, the national total cost related with the treatment of pressure ulcers in one year may be divided based on its principal components: nursing time, materials, and extra cost due to extra stays in hospitals or socio-sanitary centers. The estimated total yearly cost is 461 million €, where 22,7% correspond to primary attention, 26,2% correspond to specialized attention, and 51,1% correspond to socio-sanitary institutions. The cost per episode was determined to be 58,3 € for stage I, and 4868 € for stage IV. This represents 0,92% of the total sanitary expenditure in our country, which is estimated in 50.053,25 million euro in the year 2005.

The European Pressure Ulcer Advisory Panel and the National Pressure Ulcer Advisory Panel define pressure ulcer as an injury localized on the skin and/or lower tissue, generally speaking on a bone protrusion, as a result of pressure, or pressure in combination with shear stress. A number of factors are associated with pressure ulcers; however, the importance of such factors has not been elucidated yet. It is estimated that 95% of pressure ulcers are evitable; it is therefore important to integrate education and prevention strategies into multidisciplinary clinical practice guides taking into account the different attention levels.

The apparition of pressure ulcers is a frequent complication in immobilized patients of hospitals having a negative impact for the health of the patient, and it frequently causes the extension of the stay in the hospital and an increase in the cost of the attention. Among the different measures taken for preventing pressure ulcers, we could mention those intended to improve skin care. Dry skin is considered an independent and significant risk factor for the development of pressure ulcers. Therefore, a skin in optimal conditions will be more resistant to certain external situations. Among the measures presented in the General Guidelines for Preventing Pressure Ulcers developed by the National Group for Studying and Advising Pressure Ulcers and Chronic Wounds in 2003 in Logroño, an emphasis can be made on measures for the general care of the skin, such as the use of moisturizing creams and the use of products having hyperoxygenated fatty acids in zones under risk of developing pressure ulcers whose skin is intact, as well as protecting the skin from excessive humidity and controlling and managing pressures. However, the European Pressure Ulcer Advisory Panel and the National Pressure Ulcer Advisory Panel in 2009 recommend the use of skin emollients for moisturizing dry skin and thereby reducing the risk of skin damage, with no specific reference to hyperoxygenated fatty acids.

Pressure ulcers require the apparition of microcirculation disorders in support zones of the body located on a hard surface. That is the reason why pressure ulcers appear more frequently in areas having bone protrusions. The pressure applied on the skin and the soft tissues produces an increase of the inner pressure that causes the obstruction of blood vessels (with the formation of micro-clots) and lymphatic vessels, which in turn cause autolysis and accumulation of metabolic-toxic residues. Prolonged local ischemia causes necrosis and subsequent tissue ulceration, both on the skin as well as in deeper levels. The hydrostatic pressure of the skin capillary vessels is about 16 to 32 mmHg. Any pressure exceeding these figures causes the blood flow decrease and may cause ischemic damage even in less than two hours.

Hyperoxygenated fatty acids are products formed by essential fatty acids which have been subjected to an hyperoxygenation process. They are used externally for preventing pressure ulcers.

In the present application, a "vascular ulcer" is an injury where a loss of substance, epithelium and/or conjunctiva has occurred due to a pathologic process having a vascular origin; they follow a chronic evolution and have little or no tendency towards spontaneous cicatrization. They are painful injuries which make walking difficult and alter the patient's own physical image; pain is frequent and it may even be serious, continuous or incapacitating. They are chronic and recurrent with a high recurrence rate. They are classified as venous or arterial ulcers. In particular, venous ulcers are injuries occurring generally in the legs and most frequently in elderly persons. They are the biggest cause of chronic injuries, approximately between 70% and 90% of the cases. There are multiple theories as to their origin, although it probably has a number of causes. Currently, the most widely accepted theories consider that they appear on a pre-ulcerous base of an ischemic process. Thus, as a venous hypertension develops, high molecular weight proteins would be exuded towards the outside of the vessels, along with the extravasation of red cells or following small local hemorrhages. These proteins would be subject to organization, as is the case with fibrinogen turning into fibrin, or else they would neutralize other proteins, as is the case with alfa-2-macroglobuline that inhibits growth factors. Also, the venous hypertension would cause an accumulation of leucocytes en local thrombosis in the venules. These physio-pathological processes occur repeatedly with age. All of it would result in the existence of a zone around the vessels with a low content of growth factors, the lack of which would cause a lack of regeneration of the tissues once the protective ability of the epidermis is lost. Therefore, the re-epithelization and creation of new dermis would be altered as a consequence of such a state in elderly persons when breakage of the skin occurs.

Oil, such as olive oil, seed oil, pot marigold oil or lavender oil, have been widely employed as moisturizers, conditioners or cleaners of the skin. Olive oil is rich in essential fatty acids such as oleic and linoleic acids, phytoesteroles and squalene. All these molecules make of olive oil an exceptional conditioning, substantive and overgreasing agent capable of carrying out a protective, emollient and restorative action, while at the same time providing elasticity and a healthy look to the skin along with a pleasant and smooth feeling when touched. It has a protective activity which is useful in solar products, it shows painkilling and repithelizing properties in the treatment of erythema in after sun preparations.

Multiple pharmaceutical and cosmetic preparations have olive oil in their composition. Specifically, multiple products sold in pharmacies include olive oil, such as baby bath foams and moisturizing bath gels.

Nowadays, there is scientific evidence showing that the hyperoxygenated fatty acids are effective in the described processes. However, they are still non refundable products by the social security service, and the cost of the treatment is high.

In a study for assessing material resources for the prevention and treatment of pressure ulcers, moisturizing milk or cream is described as the most widely used product in multiple centers and hospitals, followed by hyperoxygenated fatty acids, which are currently employed in hospitalization units but not in geriatric residences. Moisturizing milks or creams are used in 100% of the residencies in contrast with just 18,2% of hyperoxygenated fatty acids.

Extra virgin olive oil is composed mostly by an oily fraction made by fatty acids, mostly oleic, palmitic and linoleic acid. Another minor non oily where, among other compounds, vitamin E and polyphenols, having an antioxidant action on the cellular membrane, can be found.

There are studies proving that not only oleic acid is responsible for the healthy effects of olive oil, since these effects are also caused by phenolic compounds. The concentration of polyphenols in extra virgin olive oil is between 330 and 500 mg/kg.

On the other hand, fatty acids are molecules formed by a long hydrocarbon chain with a pair of carbon atoms and in whose end there is a carboxyl group. Each carbon atom is bonded to the next one and to the previous one by means of a simple covalent bond. The end atom has three free positions to which hydrogen atoms are bonded. The remaining atoms have two free positions to which hydrogen atoms are also bonded.

The oxidation of fatty acids takes place in the double bonds, the oxidation speed increases with the contact with oxygen and light, the peroxide level rising. Subsequently, the decomposition of the hidroperoxides generates volatile products such as aldehydes, carboxylic acids, ketones, alcohols, etc.

The peroxide index is an index measuring the initial oxidation state of fatty acids; it is expressed in terms of miliequivalents of active oxygen by kilogram of fat, and it indicates the rancidity degree of oil. The highest the peroxide index, the lowest the antioxidant capability of oil.

The main function of oil is its antioxidant action; therefor, oil must not have many peroxides. The law allows up to 20 mEq/kg of fat (Royal Decree 308/1983 of January 25^{th} whereby the Technical Sanitary Edible Vegetable Oil regulation is approved and CEE Regulation N° 2568/91 of the Commision of July 11^{th} 1991). The peroxides are generally speaking toxic compounds. Linoleic acid hydroperoxides are among the most toxic peroxides caused by fat alteration and, in general, they alter vitamins and hemoglobin, they inhibit some enzymes, they can spark a mutagenic action, they can also produce pathologic injuries in the digestive system, and they are believed to sensitize the action of certain carcinogenic agents. Cortesi et al (Lipids, 1992; 27: 715-721) have shown a great toxicity in rats administered with intravenous dose of hydroperoxides.

The composition of hyperoxygenated fatty acids contains between 40 and 50 mg of polyphenols per kg of fat, and between 230 and 340 miliequivalents of peroxide per kg of fat. Extra virgin olive oil has between 330 and 500 mg of polyphenols per kg of fat, and less than 20 miliequivalents of peroxide per kg of fat.

Due to the difficulty to access the hyperoxygenated fatty acids and to the chemical processes oil and fatty acids are subject to when being hyperoxygenated, it is necessary to find an alternative solution for the treatment and prevention of pressure ulcers.

### DESCRIPTION OF THE INVENTION

The authors of the present invention have studied the therapeutic effect of olive oil, and preferably of extra virgin olive oil, obtained only by means of mechanical means, in pressure ulcers, and they have not seen any significant differences in comparison with the reference treatment, thereby providing solution that is simpler and more accessible to sanitary systems. They have proven that the components of extra virgin olive oil, such as vitamin A, which is a fat-soluble vitamin involved in the creation of soft and bone tissues, of mucous membranes and of the skin; tocopherols such as vitamin E, having an antioxidant effect on the cellular membrane, clorophile, esterols such as b-sitosterol and phenolic compounds such as polyphenols having an antioxidant action, as well as other components present in extra virgin olive oil, allow oil for having a beneficial effect in connection with the prevention and treatment of diseases involving an ischemic process.

In addition, the authors of the present invention have developed a composition based on non-hyperoxygenated olive oil, tocopherol, hyperic and mint, and also a pharmaceutical preparation in a pressurized container allowing for the application of the product without the need to massage the zone (since massaging the bone protrusions may cause additional damage), guaranteeing the properties from the first application to the last one, since the product contained under pressure in an opaque container does not contact oxygen until its application, it is protected from the light, it allows for a full use of the product, makes the job of the nurses easier, and it decreases the risk of bacterial contamination.

Therefore, a first aspect of the invention refers to a composition, in the following the first composition of the invention, comprising:
a. olive oil,
b. tocopherol, and
c. hyperic

The composition, therefore, comprises olive oil, it contains hyperic, and also an added additional quantity of tocopherol. Additionally, the authors of the present invention have added a substance for providing a pleasant scent and for promoting other properties of the composition. Therefore, in a more preferred embodiment, the composition comprises mint.

In another preferred embodiment of this aspect of the invention, olive oil is extra virgin olive oil obtained only by mechanical means and not hyperoxygenated. In another preferred embodiment, the proportion of olive oil is between 85 and 98%, more preferably between 90 and 97%, and even more preferably in a proportion of about 95%. In another preferred embodiment, the proportion of tocopherol, hyperic acid and mint is between 2 and 15%, more preferably between 3 and 10%, and even more preferably in a concentration of approximately 5%.

In the present application, the term "tocopherol" comprises several organic compounds formed by various methyl phenols, which form a class of chemical compounds called tocopherols, some of which act as vitamin E. In a preferred embodiment, tocopherol is selected from the list comprising α-tocopherol, β-tocopherol, γ-tocopherol, δ-tocopherol, any combination thereof, or any of their salts, prodrug, derivatives or analogues, or any of their combinations. As used herein, the term "derivative" includes pharmaceutically acceptable compounds, that is, derivatives from α-tocopherol, β-tocopherol, γ-tocopherol, δ-tocopherol which can be employed for producing a medicament, as well as non pharmaceutically acceptable derivatives, since these may be useful for preparing pharmaceutically acceptable derivatives. Thus, the salts may

Also, the present invention also encompasses pro-drugs of α-tocopherol, β-tocopherol, γ-tocopherol, δ-tocopherol. The term "pro-drug" as used herein includes any compound obtained from α-tocopherol, β-tocopherol, γ-tocopherol, δ-tocopherol, for example esters, including carboxylic acid esters, amino acid esters, phosphate esters, metallic salt sulphonate esters, etc., carbamates, amides etc., which, when administered to an individual is capable of providing, directly or indirectly, said α-tocopherol, β-tocopherol, γ-tocopherol, δ-tocopherol to said individual. Advantageously, said derivative is a compound that increases the bio-availability of α-tocopherol, β-tocopherol, γ-tocopherol, δ-tocopherol when administered to an individual or that boosts the release of α-tocopherol, β-tocopherol, γ-tocopherol, δ-tocopherol in a biologic compartment. The characteristics of said derivative are not critical provided that it can be administered to an individual and that it provides α-tocopherol, β-tocopherol, γ-tocopherol, δ-tocopherol in a biologic compartment of the individual. The preparation of such pro-drug may be carried out by means of conventional methods known by the skilled person.

In the present application, the term "hyperic" comprises any derivative obtained from genus *Hypericum* plants, and more preferably from species *Hypericum perforatum L.,* such as, without limitation, preparations based on dye, hydroalcoholic extracts, fluid extract, either dry or hydroglycolic, or any of its main constituents or their combinations. Said derivatives are known by the skilled person, and some of them are described in the pharmacopoeias, for example, without limitation, the European Pharmacopoeia, and also in vademecums and fitotherapy manuals. More preferably, it refers to the liquid extract from *Hypericum perforatum L.* added as such to oil in the production process.

In the present application, the term "mint" comprises any derivative obtained from genus *Mentha* plants, and more preferably from species *Menta x piperita L.,* such as, for example, without limitation, essential oil, hydroalcoholic extracts, fluid extract, dry or hydroglycolic, or any of its main constituents or their combinations. Said derivatives are known by the skilled person, and some of them are described in the pharmacopoeias, for example, without limitation, the European Pharmacopoeia, and also in vademecums and fitotherapy manuals. More preferably, reference is made to the product obtained by water steam distillation from stems, leafs and floral tips of *Menta x piperita L.* In addition to a pleasant scent, mint has a plurality of generally known properties which are disclosed in the pharmacopoeias and which improve the composition.

Another aspect of the invention refers to the use of olive oil for producing a medicament for the treatment and prevention of diseases involving an ischemic process, or alternatively, to olive oil for its use in the treatment and prevention diseases involving an ischemic process. In a preferred embodiment of this aspect of the invention, the disease involving an ischemic process is selected from the list consisting of pressure ulcers, vascular ulcers, diabetic foot ulcers, or any of their combinations. In another preferred embodiment of this aspect of the invention, olive oil is extra virgin olive oil, obtained only by mechanical means.

Another aspect of the invention refers to the use of a composition, in the following the second composition of the invention, comprising
a) olive oil, or
b) the first composition of the invention,
for producing a medicament for the treatment and prevention of diseases involving an ischemic process, or alternatively, a composition comprising a) olive oil, or b) the first composition of the invention, for its use in the treatment and prevention of diseases involving an ischemic process. In a preferred embodiment of this aspect of the invention, the disease involving an ischemic process is selected from the list consisting on pressure ulcers, vascular ulcers, diabetic foot ulcers, or any combination thereof. In another preferred embodiment of this aspect of the invention, olive oil is extra virgin olive oil, obtained only by mechanical means.

Said compositions may be pharmaceutical compositions. Therefore, another aspect of the invention refers to the use of a pharmaceutical composition, in the following the second composition of the invention, comprising a) olive oil, or b) the first composition of the invention for producing a medicament for the treatment and prevention of diseases involving an ischemic process, or alternatively, to a pharmaceutical composition comprising a) olive oil, or b) the first composition of the invention, for its use in the treatment and prevention of diseases involving an ischemic process. In a preferred embodiment of this aspect of the invention, the disease involving an ischemic process is selected from the list consisting of pressure ulcers, vascular ulcers, diabetic foot ulcers, or any combination thereof. In another preferred embodiment of this aspect of the invention, the olive oil is extra virgin olive oil, obtained only by mechanical means.

The compositions of the invention may also be cosmetic compositions. Therefore, the cosmetic compositions of the invention may be used for treating perilesional skin. Perilesional skin is the skin around a wound. Its extension and aspect will depend on the localization, size, type and state of the ulcer. The concept of "perilesion" should adjust to the surface located visually around the wound.

The compositions of the present invention may be formulated for being administered to an animal, and more preferably to a mammal, including humans, in a variety of known forms known in the art. Therefore, without limitation, the compositions may be in an sterile aqueous solution or in a biological fluid, such as serum. The aqueous solutions may or may not be buffered, and they comprise additional active and inactive components. The additional components include salts for controlling the ionic strength, preservatives including, without limitation, antimicrobial agents, antioxidants, quelants, and the like, and nutrients including glucose, dextrose, vitamins and minerals. The compositions may be combined with various inert carriers or excipients, including, without limitation, binders such as microcrystaline cellulose, tragacanth rubber, or gelatin; excipients such as starch or lactose; dispersing agents such as alginic acid or corn starch; lubricant agents such as magnesium stearate slipping agents such as colloidal silicon dioxide; sweetening agents such as sucrose or saccharin; or aromatic agents such as mint or methyl salicilate.

Therefore, in a preferred embodiment of this aspect of the invention, the pharmaceutical composition may additionally comprise a pharmaceutically acceptable carrier. In another preferred embodiment, the pharmaceutical composition additionally comprises pharmaceutically acceptable excipients. In another preferred embodiment, the pharmaceutical composition may additionally comprise another active principle.

In another preferred embodiment of the invention, the cosmetic composition of the invention may comprise cosmetically acceptable carriers. Said carriers are known to the skilled person. In another preferred embodiment, the cosmetic composition may additionally comprise another active principle.

In the present application, the term "medicament" makes reference to any substance used for the prevention, diagnosis, relief, treatment or cure of diseases in humans or animals. In the context of the present invention, the disease causes an ischemic process, and it is preferably a pressure ulcer, vascular ulcer, diabetic foot, or any combination thereof.

As used herein, the term "active principle", "active substance", "pharmaceutically active substance", "active ingredient", or "pharmaceutically active ingredient" makes reference to any component potentially providing a pharmacologic activity or another different effect in the diagnosis, cure, relief, treatment, or prevention of a disease, or affecting the structure or function of the body of humans or animals. The term includes those components promoting a chemical change in the production of the drug and which are present therein in a modified form that provides the specific activity or effect.

The "extra virgin olive oil" is oil obtained from the fruit of an olive tree only by mechanical means or by other physical means under conditions, specially thermal conditions, which do not cause an alteration of the oil, not having suffered any treatment besides cleaning, decanting, centrifuging and filtering. Its maximum free acidity expressed in oleic acid amounts to 1 gram per 100 grams.

The peroxide index, which is the quantity (expressed in miliequivalents of active oxygen per kg of fat) of peroxides in the sample causing oxidation of potassium iodine, must be ≤ 20.

The composition of extra virgin olive oil depends on a saponifiable fraction and a non saponifiable fraction.

The saponifiable fraction represents between 96-98%. This fat is composed mostly by triglyceride, formed by free fatty acids and phospholipids.

As to the fatty acids forming part of extra virgin olive oil, oleic acid represents the greatest proportion (70-75%) (C18:1), 11,5% of palmitic acid (C16:0) and 7,5% of linoleic acid (C18:2), in addition to other fatty acids.

Composition in fatty acids obtained by gaseous phase chromatography (% m/m of methyl esters)
- Myristic acid < 0,05
- Palmitic acid 7,5 - 20,0
- Palmitoleic acid 0,3 - 3,5
- Heptadecanoic acid < 0,3
- Heptadecenoic acid < 0,3
- Estearic acid 0,5 - 5,0
- Oleic acid 55,0 - 83,0
- Linoleic acid 3,5 - 21,0
- Linolenic acid < 1,0
- Arachidic acid < 0,6
- Gadoleic (eicosenoic) acid < 0,4
- Behenic acid < 0,2*
- Lignoceric acid < 0,2

The unsaponifiable fraction represents 1,5% of the total weight. It is formed by terpenes such as b-carotene or vitamin A, which is a fat soluble substance contributing to the formation of soft and bone tissues, mucous membranes and the skin; tocophenols such as vitamin E, having an antioxidant effect on the cellular membrane, chlorophyll, sterols such as b-sitosterol and phenolic compounds such as polyphenols having an antioxidant action, prevent cellular ageing and also the formation of cancer substances.

This insaponifiable fraction is present in virgin olive oil, but not in refined olive oil.

Another aspect of the invention refers to a pharmaceutical form, in the following the pharmaceutical form of the invention, comprising the second composition of the invention. In a preferred embodiment of this aspect of the invention, the pharmaceutical form of the invention is selected from the list comprising: poultice, ointment, paste, cream, solution, suspension, emulsion, lotion, liniment, gel, hydrogel, hydrocolloid, foam, powder, or any combination thereof. In a more preferred embodiment, the pharmaceutical form of the invention is a poultice. In a much more preferred embodiment of the invention, the pharmaceutical form of the invention is selected from a solution, a solution, a suspension or an emulsion.

In the present application, the term "pharmaceutical form" makes reference to a mix of one or more active principles, with or without additives, whose physical characteristics are suitable for its dosage, preservation, administration and bioavailability.

A "poultice" or "plaster" is a pharmaceutical form consisting of a solid or semisolid form containing the active principle or principles, as well as additives, extended on a piece of fabric, plastic or adhesive tape acting as a support and protection, further having an occlusive effect and a macerating action allowing direct contact with the skin, and it softens due to the body temperature.

An "ointment" is a pharmaceutical form consisting of a preparation having a soft consistency containing the active principle or principles and additives incorporated to a suitable base providing the consistency and mass. When applied, it adheres to the skin and mucus. This base may be fat-soluble or water-soluble, it is generally anhydrous or with a maximum content of 20% water. It is also called hydrophilic ointment when the base is washable or removable with water.

A "paste" is a pharmaceutical form consisting of a semi-solid form containing the active principle or principles and additives, made from a high concentration of insoluble powder (20 to 50 percent) in oily or aqueous bases, weak absorbent or abrasive combined with soap.

A "cream" is a pharmaceutical form consisting of a liquid or semi-solid preparation containing the active principle or principles and additives required for obtaining an emulsion, generally oil in water, with a water content over 20 percent.

A "solution" is a pharmaceutical form consisting of a liquid, transparent and homogeneous liquid obtained by dissolving the active principle or principles and the additives in water, and which is employed for external or internal use. In the case of injectable, ophthalmic and otic solutions, they must be sterile. The term "solution" includes dilutions.

A "suspension" is a pharmaceutical form consisting of a disperse system formed by two phases which contain the active principle or principles and the additives. One of the phases, which is continuous or external, is generally a liquid or a semisolid, and a dispersed or internal phase is made by insoluble solids (active principles) which are dispersable in the external phase. In case it is injectable, it must be sterile.

An "emulsion" is a pharmaceutical form consisting of an heterogeneous system generally formed by two immiscible liquids, where the dispersed phase is formed by small globules distributed in a carrier in which they are immiscible. The dispersed phase is known also as the internal phase and the dispersion medium is known as the external or continuous phase. There exist emulsions like water/oil, or oil/water, which may be semi-solid or liquid. The active principle or principles and additives may be in the external or in the internal phase.

A "lotion" is a pharmaceutical form which can be in the form of a solution, a suspension or an emulsion, containing the active principle or principles and additives, where the dispersing agent is generally water.

A "liniment" is a pharmaceutical form consisting of a liquid presentation, a solution or an emulsion, containing the active principle or principles and additives, where the carrier is aqueous, alcoholic or oily.

A "jelly" is a pharmaceutical form consisting of a semi-solid colloid containing the active principle or principles and additives, having a water-soluble base generally made by gums such as tragacanth gum; other bases are: glycerin, pectin, alginate, glycerin boron compounds, synthetic derivatives, or natural substances such as carboxymethyllcelulose.

A "gel" is a pharmaceutical form consisting of a semi-solid preparation containing the active principle or principles and additives, solid in a liquid such as water, alcohol or oil, such that the particles form a net trapped inside the liquid phase.

An "hydrogel" is a system in a colloidal state having a solid appearance, such as albumin coagulated by heat, gelatin gelled by cold, etc. A property of hydrogels is that they swell and increase their volume when the absorb water and substances dissolved therein, this property being common to all tissues of organisms formed by colloidal matter.

A "colloid" is a material formed by a dispersed phase (internal) and a dispersing phase (filler). When the dispersing phase is water, it is called "hydrocolloid". They can coagulate (go from solution to solid gel) if the dispersing phase is abundant, and flocculate (go from gel to solution) when the dispersion phase is scarce.

A "foam" is a pharmaceutical form consisting of a semi-solid preparation formed by two phases: a liquid phase carrying the active principle or principles and additives, and a gaseous phase comprising a propulsion gas for causing the product to exit in the form of a cloud.

Another aspect of the invention refers to the use of the second composition or of the pharmaceutical form of the invention for producing a medicament, or alternatively, to the second composition or the pharmaceutical form of the invention for its use as a medicament.

The correct dosage for providing a therapeutically effective quantity depends on various factors, such as age, weight, sex, tolerance,... of the mammal. As employed in this description, the expression "therapeutically effective quantity" refers to the quantity of olive oil producing the desired effect, and generally speaking it will be determined, among other causes, by the olive oil's own characteristics and by the desired therapeutic effect. "Adjuvants" and "pharmaceutically acceptable carriers" suitable for those compositions are known by the skilled person.

Another aspect of the present invention refers to a pharmaceutical preparation comprising the pharmaceutical form of the invention. More preferably, it is a pharmaceutical preparation in a pressurized container, even more preferably the pharmaceutical form is selected between a solution, a suspension or an emulsion, and even more preferably the propulsion gas is selected between isobutane, propane, butane, or any combination thereof.

The pharmaceutical preparations in a pressurized container are preparations enclosed in special containers which are pressurized by a gas. They contain one or more active principles. They are released from the container by means of a suitable valve in the form of spray (dispersion of solid or liquid particles in a gas, the size of the particles being adapted to the intended use) or a liquid or semiliquid stream, such as a foam. The pressure required for the propulsion of the preparation is generated by means of suitable propulsion gases. They are solutions, emulsions or suspensions. They are intended for local application on the skin, on the mucus of various body cavities or for inhalation. Suitable excipients may be employed, such as for example solvents, solubilizers, emulsifiers, suspension agents, and lubricants intended to prevent the valve from clogging.

In the present specification, a "pharmaceutical form" is any substance or mix of substances made, sold, offered for sale, or presented as useful for the following purposes:
a) the diagnostic, treatment, relief, or prophylaxis of a disease, of a physical anomaly or of the symptoms of one or the other in humans or animals.
b) the recovery, correction or alteration of organic functions in humans or animals.

Propulsion gases. They are pressurized liquefied gases, compressed gases or low boiling point liquids. Liquefied gases may be, for example, fluorine hydrocarbon and low molecular weight hydrocarbon (such as propane or butane). Some of the compressed gases are carbon dioxide, nitrogen and nitrous oxide. Saturated hydrocarbons such as isobutane, propane and butane are advantageous in that they are inert and have a low effect on the ozone layer.

It is also possible to mix various propulsion gases for obtaining optimal properties in terms of solubility and suitable characteristics as to pressure, expulsion and pulverization.

Containers. They are watertight and withstand internal pressure. They may be made of metal, glass, plastic, or any combination thereof. They are compatible with the preparation contained therein. Glass containers are protected by a plastic material lining.

Pulverization devices. The valve keeps the container hermetically closed while not used and regulates the delivery of the product when in use. The characteristics of the pulverization depend on the type of pulverization device, and in particular on the size, number, and location of the orifice or orifices. Certain valves provide a continuous distribution, others (known as dosing valves) release a predetermined quantity of product each time the user pushes. The materials forming the valve which are in contact with the preparation contained in the container must be suitable for that purpose.

The last aspect of the invention refers to the use of extra virgin olive oil, of the first composition of the invention, of the second composition of the invention, of the pharmaceutical composition of the invention or of the cosmetic composition of the invention, of the pharmaceutical form of the invention, or of the pharmaceutical preparation of the invention, for producing a medicament for the prevention and treatment of pressure ulcers.

In the description and claims, the term "comprise" and any variants thereof do not intend to exclude other technical features, additives, components or steps. For the skilled person, other objects, advantages and characteristics of the invention will be derived in part from the description and in part from the practice of the invention. The following examples and drawings are provided as an illustration, and they are not intended limit the scope of the present invention.

### BRIEF DESCRIPTION OF THE DRAWINGS

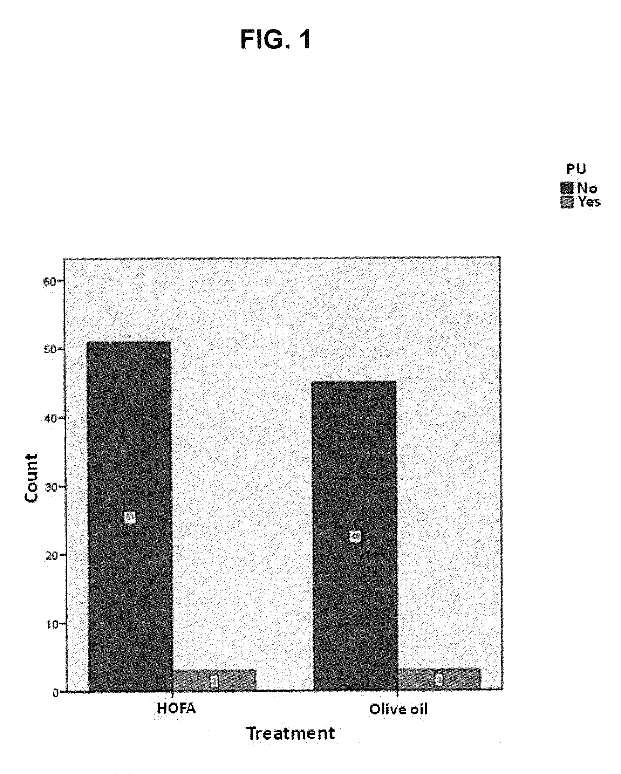

Fig. 1. Shows a comparison between the results of the treatment with the tested composition and the results of the reference treatment with hyperoxygenated fatty acids (HOFA).

### EXAMPLES

Next, the invention is disclosed by means of tests carried out by the inventors showing the effectiveness of the composition of the invention for the treatment of pressure ulcers. The following specific examples provided in the present patent document disclose the present invention. These examples are included merely for illustrative purposes, and they should not be interpreted as limiting the scope of the claimed invention. Therefore, the examples disclosed below disclose the invention without limiting the scope of application thereof.

### EXAMPLES

### Materials and methods

### Design

A clinical, multi-center, random, controlled, and triple-blinded test was carried out.

### Subjects and scope

The population of the study was formed by residents having a moderate or high risk of developing pressure ulcers. Those under risk were sampled consecutively. The period of study was from January 1^{st} 2011 to December 31^{st} 2011.

The test began in residence Inmaculada Concepción, in Puente Genil. Subsequently, those residences located in the province of Cordoba and interested in participating joined the study until the sample size was completed. The participating residencies were: Inmaculada Concepción, in Puente Genil, Hogar Residencia San Rafael, in Montilla, geriatric residence Personalia, in Baena, residencia Hospital San Sebastión, in Palma del Rio, municipal residence Guadalquivir de Córdoba, and residence SAR QUAVITAE Remedios, in Aguilar de la Frontera.

The inclusion criteria were: showing a moderate or high risk of developing pressure ulcers according to the Braden scale with a score equal or lower than 14, and not showing at the beginning of the test any non-paling erythema, grade I. Patients which, at the beginning of the test, were in an extremely serious situation, had pressure, vascular or diabetic ulcers, suffered any type of vascular disease, and those not agreeing to participate in written, were excluded from the study.

### Variables

The main result variable was the absence of ulcers (defined as not presenting an erythema which does not go pale with pressure). The security of the treatment was assessed in view of the absence of adverse effects. Also, further variables were collected such as demographic variables, risk diagnosis (Braden scale), base pathology (hypertension, diabetes, dementia, and others), usual posture (bed, bed-armchair, wandering), posture changes (the usual frequency for posture changes is every two hours, sometimes excluding sleeping hours, that is, at least 6 or more changes a day), localization of the zones of risk, use of local pressure relief surfaces (cushions, heel cushions, protections), use of specially designed surfaces for handling pressure (fixed or alternating pressure air mattresses), incontinence (urinary, fecal or total), delivery of food supplements, and time until the event in case a pressure ulcer appears.

The intervention consisted, in addition to the usual measures for preventing pressure ulcers, in applying olive oil on zones of risk (heels, malleolus, femoral condyles, greater trochanter, gluteus, sacrum, elbows, scapula and shoulders) in the experimental group, while hyperoxygenated fatty acids were applied in the control group. Both products arrived at the residence in identical containers as to shape, size, color and smell, and they were identified by means of a numeric code. The product was applied using a spray, two pulverizations on the zones of risk twice a day, every 12 hours as recommended by prior studies, one during morning hygiene and a second one before going to sleep. After the pulverizations on the zone of risk, depending on the surface thereof, the droplets were extended using the fingertips without friction. The study was left when an erythema or an adverse reaction appeared, when a patient so requested, or when the base pathology got worse. The patients and/or relatives, the nurses and the investigators did not know the product they were using until data analysis ended. The observation took place during a 30 days period.

Extra virgin olive oil was used because it contains a higher level of antioxidants, in particular phenols and vitamin E, and because it's been less processed. It was bought in a supermarket with a store brand. A scent was added in order not to be different with hyperoxygenated fatty acids. The brand of the hyperoxygenated fatty acids employed in the test own the CE marking, class IIB; this is a higher level than most products in the market obtained from hyperoxygenated fatty acids. The products were prepared and bottled by an external pharmacy.

### Methods for collecting information

The risk of developing pressure ulcers was determined before beginning the study with the aid of the Braden scale.

The initial assessment of the skin and of the risk was carried out at the beginning by the main investigator in cooperation with one of the collaborators and with the chief nurse of the residence.

Subsequently, every seven days after the beginning of the study the risk was assessed again by the main investigator and by a collaborator with the aid of the data collection sheet. The remaining evaluations were carried out by the nurse. Said evaluations were carried out daily before the application of the product. The nurses participating in the study were suitably trained in the application of the product and collection of data. The evolution of the patients was annotated in a data collection sheet specifically designed for that purpose.

### Statistical analysis

The sample size was calculated using program Epidat 3.1® starting from the incidence data corresponding to first degree ulcers in the control group, which amounts to 17,37% in contrast with 7,32% of the intervention group, starting from a previous study related with hyperoxygenated fatty acids. Therefore, the expected incidence difference will be about 10%. Accepting a risk α of 0,5 and a risk β of 0,20 in a bilateral contrast, and estimating a loss frequency of 10% in a proportion comparison for independent groups, the number of residents must be 167 per group. Two groups were determined, the experimental group with olive oil and the control group with hyperoxygenated fatty acids. The residents were randomly assigned to one of the groups using program Epidat 3.1®.

Data analysis was carried out using statistic analysis program SPSS 18.0®. After cleaning the data, a descriptive analysis per treatment group was carried out. Qualitative variables were described in terms of absolute frequencies and percentages, while the quantitative variables were described using the mean and the standard deviation (DE). The normality assumptions were checked. In order to compare the quantitative variable means per treatment group, the "t-Student" test was employed for independent samples and non parametric test "U-Mann Whitney". The association between qualitative variables was assessed by means of the "ji-squared" test, or with the exact Fisher test in case more than the expected 25% were lower than 5. The absolute difference in risks was calculated along with their IC of 95%, relative risk (RR), and necessary number to treat (NNT). Kaplan Meier survival curves were applied in order to evaluate the temporal evolution in connection with application of olive oil and hyperoxygenated fatty acids, and proportional risk models or Cox regression for comparing the risk between all variables registered for each subject along with time and the survival rate.

### Results

A total of 104 patients were included in the study, 100 of which were still present at the end, 49 (49%) in the olive oil group and 51 (51%) in the hyperoxygenated fatty acid group.

For losses were recorded, one of them due to an incomplete filling of the data collection sheet and three due to exitus.

The incidence of pressure ulcers during the period of study was 6,1% (3/49) in the olive oil group, while in the hyperoxygenated fatty acids it was 5,9% (3/51), (p≤1) (Fig. 1).

The base characteristics and a comparison between the two groups are shown in Table 1, where we see that there are differences in the mean age, being 3,8 years more for the group with olive oil. In addition, there are more women in the olive oil group.

With respect to food supplements, the olive oil group shows a higher percentage of residents employing food supplements.

The comparison of survival Kaplan-Meier curves using the log-rank test shows a statistical significance of the usual posture; this is shown along with the accumulated incidence at day fifteen and thirty in Table 2.

No adverse effects appeared in connection with the external application of extra virgin olive oil on healthy skin.

**Table 1 Base characteristics of the treatment groups**

| | Olive oil group | HOFA group | P |
|---|---|---|---|
| | n= 49 (47,6%) | n=54 (52,4%) | |
| Age* | 85,12 (7,1) | 81,28 (9,27) | 0,21 |
| Braden* | 12,04 (1,44) | 12,07 (1,55) | 0,91 |
| Gender | | | 0,073 |
| Men | 8 (16,3%) | 17 (31,5%) | |
| Women | 41 (83,7%) | 37 (68,5%) | |
| | | | |
| Risk | | | 0,632 |
| Moderate | 24 (49%) | 29 (53,7%) | |
| High | 25 (51%) | 25 (46,3%) | |
| | | | |
| Incontinence | | | 0,328 |
| Simple | 3 (6,3%) | 7 (13%) | |
| Mixed | 45 (93,8%) | 47 (87%) | |
| | | | |
| Usual posture | | | 0,619 |
| Bed | 1 (2,1%) | 3 (5,7%) | |
| Bed-Armchair-Wheelchair | 47 (97,9%) | 50 (94,3%) | |
| | | | |
| Posture changes | | | 0,174 |
| YES | 24 (50%) | 19 (36,5%) | |
| NO | 24 (50%) | 33 (63,5%) | |
| | | | |
| Pressure managing local devices | | | 0,98 |
| YES | 30 (62,5%) | 33 (62,3%) | |
| NO | 18 (37,5%) | 20 (37,7%) | |
| | | | |
| Pressure managing special surfaces | | | 0,145 |
| YES | 27 (55,1%) | 22 (40,7%) | |
| NO | 22(44,9%) | 32 (59,3%) | |
| | | | |
| Food supplements | | | 0,006 |
| Yes | 9 (18,8%) | 1 (1,9%) | |
| NO | 39 (81,3%) | 52 (98,1%) | |
| | | | |
| *standar deviation mean (DE) | | | |

**Table 2. Survival table after 15 and 30 days**

| **Variable** | **Survival 15 days** | **Survival 30 days** | **P (log-rank)** |
|---|---|---|---|
| **GENDER** | | | 0,706 |
| Man | 95,8% | 95,8% | |
| Woman | 98,7% | 93,4% | |
| **AGE** | | | 0,99 |
| <75 years | 94,1% | 94,1% | |
| 75-85 | 100% | 93,8% | |
| >85 | 98,1% | 94,1% | |
| **RISK** | | | |
| Moderate | 98,1 % | 92,3% | 0,465 |
| High | 97,9% | 95,8% | |
| **INCONTINENCE** | | | 0,544 |
| Simple | 90% | 90% | |
| Mixed | 98,9 | 94,4% | |
| **USUAL POSTURE** | | | 0,76 |
| Bed | 66,7% | 66,7% | |
| Bed-Armchair-Wheelchair | 99% | 94,7% | |
| **POSTURE CHANGES** | | | 0,705 |
| YES | 97,6% | 92,7% | |
| NO | 98,2% | 94,7% | |
| **PRESSURE MANAGING LOCAL DEVICE** | | | 0,53 |
| YES | 100% | 95% | |
| NO | 97,4% | 92% | |
| P**RESSURE MANAGING SPECIAL SURFACES** | | | 0,31 |
| YES | 97,9% | 91,4% | |
| NO | 100% | 96,2% | |
| **FOOD SUPPLEMENTS** | | | 0,615 |
| YES | 100% | 90% | |
| NO | 98,9% | 94,4% | |
| **TREATMENT** | | | |
| Olive oil | 100% | 93% | |
| Hyperoxygenated fatty | 98,1% | 94,3% | 0,2 |
| acids | | | |

**Table 3. Univariate analysis**

| **Variable** | **Univariate analysis RR(IC 95%)** | **P** |
|---|---|---|
| **GENDER** | | |
| | | |
| **AGE** | | |
| 75-85 | 1,037 (0,094-11,43) | 0,976 |
| >85 | 0,99 (0,103-9,54) | 0,995 |
| | | |
| **RISK** | 0,537 (0,098-2,934) | 0,473 |
| | | |
| **INCONTINENCE** | 0,722 (0,247-2,11) | 0,551 |
| | | |
| **USUAL POSTURE** | 0,131 (0,015-1,136) | 0,065 |
| | | |
| **POSTURE CHANGES** | 1,35 (0,274-6,73) | 0,707 |
| | | |
| **PRESSURE MANAGING LOCAL DEVICES** | 0,603 (0,122-2,98) | 0,535 |
| | | |
| **PRESSURE MANAGING SPECIAL SURFACES** | 2,33 (0,428-12,75) | 0,327 |
| | | |
| **FOOD SUPPLEMENTS** | 1,72 (0,201-14,74) | 0,620 |
| | | |
| **TREATMENT** | 1,075 (0,217-5,32) | 0,93 |

### Discussion

The main limitation of the study was related with the sample size, since this type of study requires a great number of patients in risk of developing a pressure ulcer; that is the reason why the study took three years. The geographic dispersion of the residences made travelling necessary in order to carry out the assessments and data collection. Another limitation of this study is related with the losses due to residents passing away or moving out of the residence.

The possible bias in connection with the application of the product by different people was controlled because the personnel involved in the study did not know the product being applied. In order to avoid any information bias, the main investigator will be the only evaluator, accompanying the nurses of the geriatric residencies involved in the study when the skin assessment takes place.

The cost-benefit variable was not assessed in this test, but taking into account that the nursing time is identical in prevention, it favors the costs in the olive oil group.

The survival curves of both treatment groups were compared with no significant differences; subsequently, the remaining variables were isolated but still no differences were found. In connection with the multivariate analysis, variable time in days was considered as the dependent variable. Gender, age in years, the risk of developing pressure ulcers, type of incontinence, usual posture, posture changes, pressure managing local devices, pressure managing special surfaces, food supplements and treatments were considered as independent variables. Variables having p ≤ 0,15 in connection with Wald statistic were eliminated in Cox regression model one by one for arriving at the reduced model. Subsequently, the initial model is compared with the likelihood reason test. When the Cox regression with the final covariables of the model of the invention was performed, no statistically significant differences were found.

### Conclusions

No statistically significant differences were found to exist between both treatments. Therefore, since there seem to be no statistical difference and since no other design may be applied, based on the data obtained during the study we can consider extra virgin olive oil as a therapeutic option in view of its innocuousness when externally applied, the reduced pressure ulcer incidence during treatment (graph 1), its antioxidant capabilities, its low peroxide content in comparison with alternative treatments, and its lower cost.

## Claims

1. A composition comprising:
a. olive oil,
b. tocopherol
c. hyperic

2. The composition according to the previous claim, further comprising mint.

3. The composition according to any of claims 1-2, where the olive oil is extra virgin olive oil.

4. The composition according to any of claims 1-3, where olive oil is found in a proportion of between 90 and 97%.

5. The composition according to any of claims 1-4, where tocopherol, hyperic acid and mint are found in a proportion of between 3 and 10%.

6. The composition according to any of claims 1-5, where olive oil is found in a proportion of approximately 95%, and tocopherol, hyperic and mint are found in a proportion of approximately 5%.

7. The composition according to any of claims 1-6, which is a pharmaceutical composition.

8. The composition according to any of claims 1-6, which is a cosmetic composition.

9. Use of the composition according to any of claims 1-7 for producing a medicament.

10. Use of:
a. olive oil, or
b. a composition according to any of claims 1-7,
for producing a medicament for the treatment and prevention of diseases involving an ischemic process.

11. Use of a pharmaceutical composition comprising:
a. olive oil, or
b. a composition according to any of claims 1-7,
for producing a medicament for the treatment and prevention of diseases involving an ischemic process.

12. The use of a pharmaceutical composition according to the previous claim, where the disease involving an ischemic process is selected from the list consisting of: pressure ulcers, vascular ulcers, diabetic foot, or any combination thereof.

13. The use of a cosmetic composition comprising:
a. olive oil, or
b. a composition according to any of claims 1-6,
for the treatment and prevention of pressure ulcers, vascular ulcers, diabetic ulcers, or any combination thereof.

14. The use according to any of claims 10-13, where olive oil is extra virgin olive oil.

15. The use of a composition according to any of claims 10-14, where the composition further comprises a cosmetically or pharmaceutically acceptable carrier.

16. The use of a composition according to any of claims 10-15, where the composition further comprises an additional active principle.

17. Pharmaceutical form comprising a composition as described in any of claims 1-16.

18. Pharmaceutical form according to the previous claim, which is selected from the list consisting of: poultice, ointment, paste, cream, solution, suspension, emulsion, lotion, liniment, gel, hydrogel, hydrocolloid, foam, powder, or any combination thereof.

19. A pharmaceutical preparation comprising a pharmaceutical form according to any of claims 17-18.

20. The pharmaceutical preparation according to the previous claim, which is a pharmaceutical preparation in a pressurized container, where the pharmaceutical form is selected between a solution, a suspension or an emulsion.

21. The pharmaceutical preparation in a pressurized container according to the previous claim, where the propulsion gas is butane.

22. The use of a pharmaceutical form according to any of claims 17-18 or of a pharmaceutical preparation according to any of claims 19-21, for producing a medicament.

23. The use of extra virgin olive oil, of a composition as described in any of claims 1-16, of a pharmaceutical form according to any of claims 17-18, or of a pharmaceutical preparation according to any of claims 19-21, for producing a medicament for the prevention and treatment of pressure ulcers.
